# EUROPEAN PATENT APPLICATION

(11) **EP 4 632 783 A1**
(43) Date of publication of application: **15.10.2025**
(21) Application number: 23900726.3
(22) Date of filing: 07.12.2023
(51) Int. Cl.: H01J 35/10, A61B 6/40, F16C 17/02, F16C 33/06, F16C 33/24, H05G 1/00, H05G 1/02

(54) **ROTARY POSITIVE ELECTRODE X-RAY TUBE**

(30) Priority: 08.12.2022 JP 2022196566
(71) Applicant: Canon Electron Tubes & Devices Co., Ltd., Otawara-shi, Tochigi 324-0036 (JP)
(72) Inventor: NAGAI, Masaya, Otawara-shi, Tochigi 324-0036 (JP); AIDA, Hiroshi, Otawara-shi, Tochigi 324-0036 (JP)
(74) Representative: Henkel & Partner mbB
(86) International application number: PCT/JP2023/043822
(87) International publication number: WO 2024/122614

(57) **Abstract**

A rotating anode X-ray tube includes a cathode (60) which emits electrons, an anode target (50) which generates X-rays upon receiving the electrons, a sliding bearing (3) including a rotor (20) coupled with the anode target (50) and extending along a rotation axis (a), a stationary shaft (10) supporting the rotor (20) so as to be rotatable, and a lubricant held between the rotor (20) and the stationary shaft (10), and a vacuum tube (70) which accommodates the cathode (60) and the anode target (50) and fixes the stationary shaft (10). The rotor (20) includes a bearing member (21) formed to extend along the rotating axis (a) and positioned to surround the stationary shaft (10). At least one of the stationary shaft (10) and the bearing member (21) are formed of tungsten carbide, silicon carbide, or titanium carbide.

## Description

### Technical Field

Embodiments described herein relate generally to a rotating anode X-ray tube.

### Background Art

Generally, X-ray tube assemblies are used as X-ray generators in medical and industrial equipment that uses X-rays to diagnose subjects. X-ray tube assembly is mounted in X-ray CT scanner and rotate around the subject. A rotating anode X-ray tube assembly equipped with a rotating anode X-ray tube (hereinafter also referred to as a "rotating anode X-ray tube") is known as an X-ray tube assembly.

### Citation List

### Patent Literatures

Patent Literature 1: JP H09-115467 A
Patent Literature 2: JP 2000-353485 A

### Summary of Invention

### Technical Problem

One of the objects of the present embodiment is to provide a rotating anode X-ray tube that can maintain excellent rotation of the rotor.

### Solution to Problem

According to one embodiment, there is provided a rotating anode X-ray tube comprising: a cathode which emits electrons; an anode target which generates X-rays upon receiving the electrons; a sliding bearing including a rotor coupled with the anode target and extending along a rotation axis, a stationary shaft supporting the rotor so as to be rotatable, and a lubricant held between the rotor and the stationary shaft; and a vacuum tube which accommodates the cathode and the anode target and fixes the stationary shaft. The rotor includes a bearing member formed to extend along the rotating axis and positioned to surround the stationary shaft. At least one of the stationary shaft and the bearing member are formed of tungsten carbide, silicon carbide, or titanium carbide.

### Brief Description of Drawings

FIG. 1 is a perspective view showing an external appearance of an X-ray CT scanner.
FIG. 2 is a cross-sectional view showing the X-ray CT scanner taken along line A-A in FIG. 1.
FIG. 3 is a front view of showing a rotating stand, an X-ray tube assembly, a cooling unit, and an X-ray detector shown in FIG. 2.
FIG. 4 is a cross-sectional view showing the X-ray tube assembly according to one embodiment.
FIG. 5 is a side view showing part of the stationary shaft shown in FIG. 4.
FIG. 6 is a cross-sectional view showing a modified example of the X-ray tube assembly of the above embodiment.

Mode for Carrying Out the Invention Embodiments will be described hereinafter with reference to the accompanying drawings. The disclosure is merely an example, and proper changes within the spirit of the invention, which are easily conceivable by a skilled person, are included in the scope of the invention as a matter of course. In addition, in some cases, in order to make the description clearer, the widths, thicknesses, shapes, etc., of the respective parts are schematically illustrated in the drawings, compared to the actual modes. Further, in the specification and drawings, corresponding elements are denoted by like reference numerals, and a detailed description thereof may be omitted unless otherwise necessary.

A rotating anode X-ray tube according to one embodiment will now be described in detail with reference to the accompanying drawings.

First, the basic concept of the embodiment will be explained.

An X-ray CT scanner comprises a rotating stand, an X-ray tube assembly, etc. The X-ray tube assembly has a rotating anode X-ray tube, which rotates together with the rotating stand. The rotating anode X-ray tube is equipped with a cathode, an anode target, a stationary shaft, a rotor and the like.

Currently, in X-ray CT scanners for the heart, the increasing in speed of rotation of the rotating stand is progressing. Specifically, an X-ray CT scanner that rotates the rotating stand at 0.2 s/r (5 rps) has been developed. Here, when the rotating stand is rotated at 0.2 s/r, the X-ray tube assembly is subjected to a centrifugal acceleration (centrifugal force) of approximately 70 G. Conventionally, the rotating stand is rotated at 0.35 s/r (approximately 2.8 rps). In this case, the centrifugal acceleration generated in the X-ray tube assembly is 23 G. That is, in the X-ray CT scanner under development, the X-ray tube assembly needs to withstand centrifugal acceleration that is approximately three times as strong. More specifically, when the X-ray tube assembly is subjected to high centrifugal acceleration, the stationary shaft may deform and come into contact with the rotor.

Further, the rotating anode X-ray tubes generate X-rays by colliding electrons generated at the cathode with the anode target at high speed. At this time, the efficiency of X-ray generation is approximately 1%, and the remaining energy becomes heat. For example, when if the input power, which is calculated as the product of the tube voltage, which determines the speed of the electrons, and the tube current, which determines the amount of electrons, is 100 kW, and the electrons are injected into the anode target for 4 seconds, the surface of the anode target where the electrons are injected is heated locally to nearly 3000°C. Here, the rotating anode X-ray tube cannot be used until the temperature of the anode target drops to a certain level, and therefore it is desirable to dissipate the heat from the rotating anode X-ray tube as quickly as possible.

In order to solve the above-provided issues, X-ray tubes using a conduction cooling method have been developed, in which the heat of the anode target is transferred to the coolant passing through the stationary shaft by heat conduction, but there are two problems as described below.
1. The heat of the anode target is transferred to the circulating water via the bearing surface, and therefore the bearing surface is heated as well. Therefore, the rotor expands in a direction away from the stationary shaft, thereby increasing the gap between the rotor and the stationary shaft. As a result, in some cases, stable rotation of the rotor cannot be achieved.
2. In high-input rotating anode X-ray tubes, generally, gallium-indium-stannum-based liquid metal is used as a lubricant for the bearings. With this configuration, when the rotor is heated, a chemical reaction occurs between the lubricant and the bearing surfaces of the rotor and the stationary shaft, and a compound is generated on the above-described bearing surfaces. Due to this compound, the gap between the stationary shaft and the rotor is narrowed, and eventually the stationary shaft and the rotor come into contact with each other.

As described above, the present embodiment aims to improve such problems, and it is possible to obtain a rotating anode X-ray tube that can maintain good rotation of the rotor.

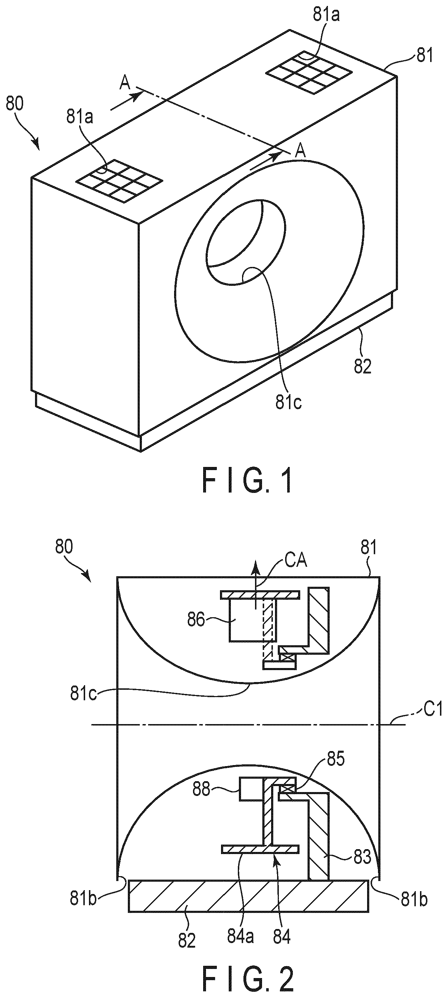

First, the configuration of an X-ray CT scanner 80 will be explained with reference to FIGS. 1 to 3.

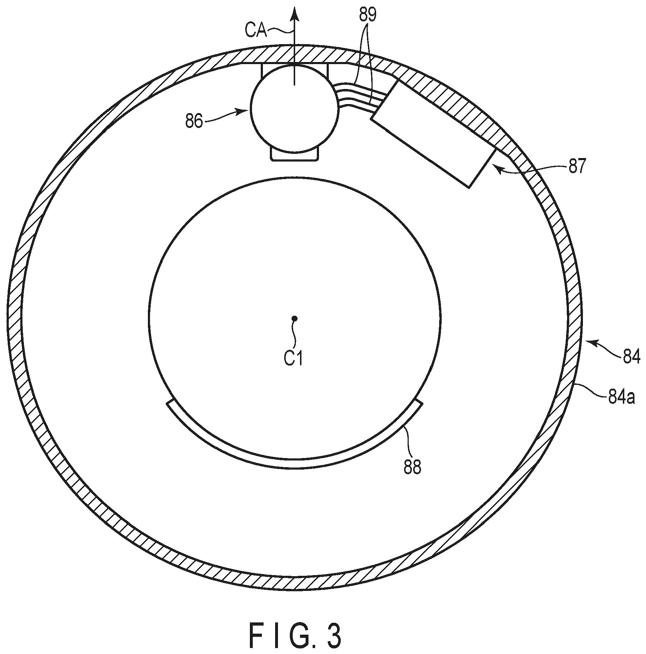

FIG. 1 is a perspective view showing an external appearance of the X-ray CT scanner 80. FIG. 2 is a cross-sectional view showing the X-ray CT scanner 80 taken along the line A-A in FIG. 1. FIG. 3 is a front view showing a rotating stand 84, an X-ray tube assembly 86, a cooling unit 87, and an X-ray detector 88 shown in FIG. 2. As shown in FIGS. 1 to 3, the X-ray CT scanner 80 comprises a housing 81, a base unit 82, a stationary stand 83, a rotating stand 84, a bearing member 85, an X-ray tube assembly 86, a cooling unit 87, and an X-ray detector 88.

The housing 81 accommodates the stationary stand 83, the rotating stand 84, the bearing member 85, the X-ray tube assembly 86 and the like. The housing 81 has an air outlet 81a, an air inlet 81b, and an introduction port 81c. The external air taken in through the air inlet 81b is exhausted through the air outlet 81a. The introduction port 81c is provided for introducing the subject. Although not shown in the figure, the X-ray CT scanner 80 comprises a bed on which the subject to be examined is placed.

The stationary stand 83 is fixed to the base unit 82. The rotating stand (, which may as well be referred to as a "gantry") 84 is rotatably supported by the stationary stand 83 via the bearing member 85. The rotating stand 84 is rotatable about a central axis C1 of the rotating stand 84. The rotating stand 84 has a ring-shaped frame unit 84a located at the outermost circumference.

The X-ray tube assembly 86, the cooling unit 87, and the X-ray detector 88 are attached to the rotating stand 84 and they rotate together with the rotating stand 84. Thus, in the X-ray tube assembly 86, centrifugal acceleration CA is generated. The X-ray tube assembly 86 and the cooling unit 87 are attached to the inner wall of the frame unit 84a. The X-ray tube assembly 86 exchanges heat with the cooling unit 87 via a circulation path 89. The X-ray tube assembly 86 functions as an X-ray generator and emits X-rays. The X-ray detector 88 detects X-rays emitted from the X-ray tube assembly 86 and passed through the subject, and converts the detected X-rays into electrical signals.

The X-ray CT scanner 80 is configured as described above.

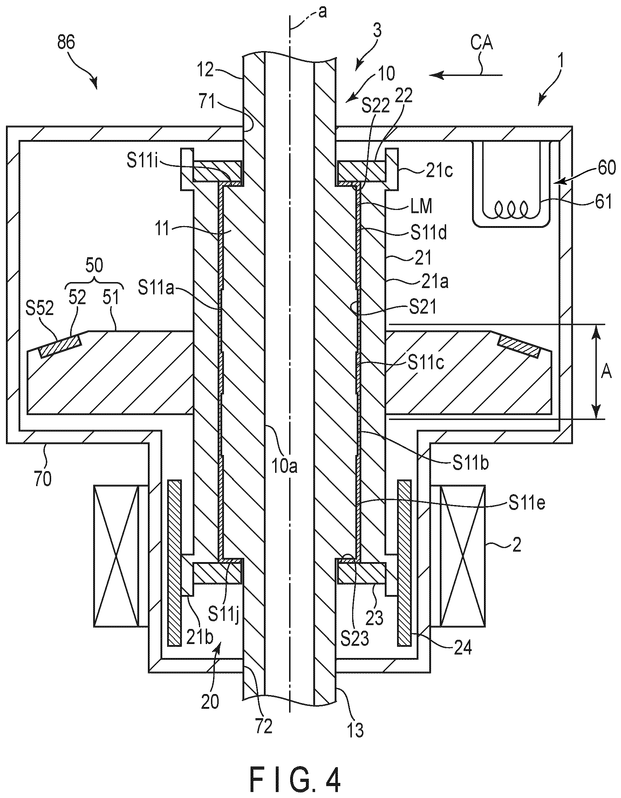

FIG. 4 is a cross-sectional view showing the X-ray tube assembly 86 of one embodiment.

As shown in FIG. 4, the X-ray tube assembly 86 comprises a rotating anode X-ray tube 1, a stator coil 2 that generates magnetic fields, and the like. The rotating anode X-ray tube 1 comprises a sliding bearing 3, an anode target 50, a cathode 60, and a vacuum tube 70. The sliding bearing 3 includes a stationary shaft 10, a rotor 20, and liquid metal LM as a lubricant.

The stationary shaft 10 is formed into a cylindrical shape, extends along a rotation axis a, and has radial bearing surfaces S11a and S11b formed on the outer circumferential surface and a heat transfer unit 10a. The stationary shaft 10 rotatably supports the rotor 20. The stationary shaft 10 is constituted by a large diameter unit 11, a first small diameter unit 12, and a second small diameter unit 13. The large diameter unit 11, the first small diameter unit 12, and the second small diameter unit 13 are formed to be integrated as one body coaxially.

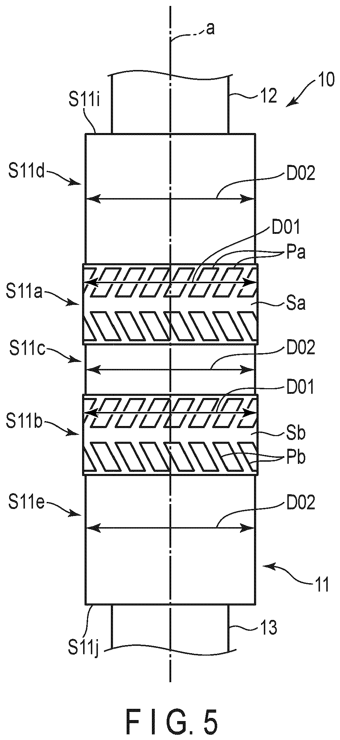

The details of the large diameter unit 11 of the stationary shaft 10 will now be described with reference to FIG. 5. FIG. 5 is a side view of a part of the stationary shaft 10 shown in FIG. 4.

As shown in FIG. 5, the large diameter unit 11 has a radial bearing surface S11a, a radial bearing surface S11b, a recessed surface S11c, a recessed surface S11d, and a recessed surface S11e, which are each located on the outer circumferential surface. Further, the large diameter unit 11 has a thrust bearing surface S11i on one end and a thrust bearing surface S11j on another end. The radial bearing surface S11a and the radial bearing surface S11b are each formed on the outer circumferential surface of the large diameter unit 11 over the entire circumference. The recessed surface S11c, the recessed surface S11d, and the recessed surface S11e are formed on the outer circumferential surface of the large diameter unit 11 over the entire circumference.

The radial bearing surface S11a and the radial bearing surface S11b are located at intervals in a direction along the rotation axis a. The recessed surface S11c is located between the radial bearing surface S11a and the radial bearing surface S11b, and is adjacent to each of the radial bearing surface S11a and the radial bearing surface S11b. The recessed surface S11d is located beyond the radial bearing surface S11a as viewed from the recessed surface S11c, and is adjacent to the radial bearing surface S11a. The recessed surface S11e is located beyond the radial bearing surface S11b as viewed from the recessed surface S11c, and is adjacent to the radial bearing surface S11b.

The radial bearing surface S11a has a plain surface Sa and a plurality of patterned units Pa. The plain surface Sa has a smooth outer circumferential surface. The patterned units Pa are formed by indenting the plain surface Sa. Each of the patterned units Pa is arranged to extend in a slanted fashion with respect to the circumferential direction. The patterned units Pa are formed at intervals in the direction along the rotation axis a. Note that the patterned units Pa may as well be connected in the direction along the rotation axis a.

The radial bearing surface S11b has a plain surface Sb and a plurality of patterned units Pb. The plain surface Sb has a smooth outer circumferential surface. The plurality of patterned units Pb are formed by indenting the plain surface Sb. The patterned units Pb are each disposed to extend in a slanted fashion with respect to the circumferential direction. The patterned units Pb are formed at intervals in the direction along the rotation axis a. Note that the plurality of patterned units Pb may be connected in the direction along the rotation axis a.

Each of the patterned units Pa and each of the patterned units Pb are formed as grooves having a depth of several tens of micrometers. The patterned units Pa and the patterned units Pb each form a herringbone pattern. Therefore, the radial bearing surfaces S11a and S11b are recessed and protruding surfaces, respectively, and can rake the liquid metal LM therein, thereby making it possible to easily generate dynamic pressure by the liquid metal LM.

The recessed surfaces S11c, S11d, and S11e are smooth outer circumferential surfaces and are plain surfaces. The recessed surfaces S11c, S11d, and S11e are formed in recesses compared to the radial bearing surfaces S11a and the radial bearing surfaces S11b. In other words, in the stationary shaft 10, the outer diameter DO2 of the zone where the recessed surfaces S11c, S11d, and S11e are formed is smaller than the outer diameter DO1, which is the minimum outer diameter of the zone where the radial bearing surfaces S11a and S11b are formed.

In the direction perpendicular to the rotation axis a, the gap between the recessed surfaces (recessed surfaces S11c, S11d, S11e) and the rotor 20 is greater than the gap between the radial bearing surface S11a (plane surface Sa) and the rotor 20, and greater than the gap between the radial bearing surface S11b (plane surface Sb) and the rotor 20.

The space between the recessed surface S11c and the rotor 20, the space between the recessed surface S11d and the rotor 20, and the space between the recessed surface S11e and the rotor 20 can be made to function as reservoirs for storing liquid metal LM. With this configuration, liquid metal LM can be supplied to each radial bearing surface S11a, S11b from both sides, and therefore the depletion of liquid metal LM in the gap between the bearings can be suppressed.

As shown in FIG. 4, the first small diameter unit 12 is formed into the shape of a solid cylinder having an outer diameter smaller than that of the large diameter unit 11, and is formed to extend out from one end of the large diameter unit 11. The first small diameter unit 12 is located on a rotation axis a side with respect to the thrust bearing surface S11i.

The second small diameter unit 13 is formed into the shape of a solid cylinder having an outer diameter smaller than that of the large diameter unit 11, and is formed to extend out from the other end of the large diameter unit 11. The second small diameter unit diameter unit 13 is located on a rotation axis a side with respect to the thrust bearing surface S11j.

The heat transfer unit 10a transfers heat to the coolant flowing inside by forced convection. The coolant is, for example, a cooling liquid L. The cooling rate of the anode target 50 of the rotating anode X-ray tube 1 can be improved by water cooling or oil cooling (insulating oil). Note that the coolant may was well be air, and the cooling rate of the anode target 50 may be improved by air cooling.

It is preferable that the heat transfer unit 10a be located in a region A that opposes at least the anode target 50. With this configuration, the part of the stationary shaft 10, which is easily heated by the anode target 50 can be cooled down.

Note here that the method of releasing heat from the stationary shaft 10 to the outside of the rotating anode X-ray tube 1 is not limited to the above-described heat transfer from the heat transfer unit 10a to the coolant, but, for example, the heat inside the stationary shaft 10 may be released by cooling the outer circumferential surface of the first small diameter unit 12 with a heat exchanger. In this case, the rotating anode X-ray tube 1 may be configured without the heat transfer unit 10a and the coolant (cooling liquid L).

The rotor 20 is coupled with the anode target 50 and extends along the rotation axis a. The rotor 20 includes a bearing member 21, a first support member 22, a second support member 23, and a tubular unit 24.

The bearing member 21 is formed in a hollow cylinder shape extending along the rotation axis a, and is located to surround the stationary shaft 10. The bearing member 21 includes a bearing member main body 21a and protrusions 21b and 21c. The bearing member main body 21a has a uniform inner diameter and a uniform outer diameter over its entire circumference, and includes a radial bearing surface S21 on its inner circumferential surface.

The protrusions 21b and 21c are formed to extend towards a direction away from the rotation axis a on both ends of the bearing member main body 21a. The protrusions 21b and 21c each have a ring-shaped configuration. The protrusions 21b and 21c may not necessarily be formed continuously and integrally with the bearing member main body 21a, and may be fixed to the outer circumferential surface of the bearing member main body 21a by welding or screws, for example.

At least one of the stationary shaft 10 and the bearing member 21 are formed of tungsten carbide (WC), silicon carbide (SiC), or titanium carbide (TiC). If the stationary shaft 10 and the bearing member 21 are not formed of tungsten carbide, silicon carbide, or titanium carbide, they are formed of SKD11, which is an alloy tool steel material specified in JIS (Japanese Industrial Standards) G 4404. Note that the material of the stationary shaft 10 and the material of the bearing member 21 may be the same. For example, both of the stationary shaft 10 and the bearing member 21 may be formed of tungsten carbide.

The Young's modulus of tungsten carbide is about 600 GPa. The Young's modulus of silicon carbide is about 450 GPa. The Young's modulus of titanium carbide is about 450 GPa. The Young's modulus of SKD11 is about 200 GPa. That is, the Young's modulus of tungsten carbide, that of silicon carbide, and that of titanium carbide are approximately 400 to 600 GPa, which are about twice the Young's modulus of SKD11.

Meanwhile, the linear expansion coefficient of tungsten carbide is approximately 5 × 10⁻⁶/K. The linear expansion coefficient of silicon carbide is 4 × 10⁻⁶/K. The linear expansion coefficient of titanium carbide is 7 × 10⁻⁶/K. The linear expansion coefficient of SKD11 is 12 × 10⁻⁶/K. That is, the linear expansion coefficients of tungsten carbide, silicon carbide, and titanium carbide are about half that of SKD11.

Further, tungsten carbide, which has a thermal conductivity of about 120 W/mK, and silicon carbide, which has a thermal conductivity of about 200 W/mK, have been developed. The thermal conductivity of SKD11 is about 20 W/mK. That is, the thermal conductivity of tungsten carbide and that of silicon carbide are higher than the thermal conductivity of SKD11.

Tungsten carbide, silicon carbide, and titanium carbide are electrically conductive. Therefore, even when tungsten carbide, silicon carbide, or titanium carbide is used for the stationary shaft 10 and the bearing member 21, a tube current can be passed through.

The first support member 22 has a circular ring shape and is fixed to one end of the bearing member 21. The first support member 22 is formed of a metal such as an Fe alloy or Mo alloy. The first support member 22 includes a thrust bearing surface S22 that opposes the thrust bearing surface S11i of the stationary shaft 10 in a direction along the rotation axis a. The thrust bearing surface S22 is located on an inner circumferential side of the first support member 22 and has a ring-shaped configuration.

The gap between the first support member 22 and the stationary shaft 10 (first small diameter unit 12) is set to a value that can maintain the rotation of the rotor 20 and suppress the leakage of the liquid metal LM as well. As described above, the gap described above is very slight, and the first support member 22 functions as a labyrinth seal ring.

The second support member 23 has a circular ring shape and is fixed to the other end of the bearing member 21. The second support member 23 is formed of a metal such as an Fe alloy or Mo alloy. The second support member 23 includes a thrust bearing surface S23 that opposes the thrust bearing surface S11j of the stationary shaft 10 in the direction along the rotation axis a. The thrust bearing surface S23 is located on the inner circumferential side of the second support member 23 and has a ring-shaped configuration.

The gap between the second support member 23 and the stationary shaft 10 (second small diameter unit 13) is set to a value that can maintain the rotation of the rotor 20 and suppress the leakage of the liquid metal LM as well. As described above, the gap is very slight, and the second support member 23 functions as a labyrinth seal ring.

The tubular unit 24 is joined to the outer circumferential surface of the protrusion 21b of the bearing member 21. The tubular unit 24 is formed of a metal such as copper (Cu) or a copper alloy.

Incidentally, when there is a difference in the thermal expansion coefficient between the bearing member 21, the first support member 22, and the second support member 23, an unwanted gap may occur between the stationary shaft 10 and the rotor 20. In order to suppress or prevent the occurrence of the above-mentioned unwanted gap, the first support member 22 and the second support member 23 may be formed of the same material as that of the bearing member 21. In that case, the first support member 22 and the second support member 23 are formed of tungsten carbide, silicon carbide, or titanium carbide.

A lubricant (the liquid metal LM in this embodiment) is retained between the rotor 20 and the stationary shaft 10. More specifically, the liquid metal LM is filled in the gaps between the stationary shaft 10 (large diameter unit 11), the bearing member 21, the first support member 22, and the second support member 23, respectively. For the liquid metal LM, materials such as gallium-indium (GaIn) alloys or gallium-indium-tin (GaInSn) alloys can be used. During the operation of the rotor 20, the liquid surface on the rotational axis a side of the liquid metal LM is positioned on the rotational axis a side with respect to the radial bearing surfaces S11a and S11b. With this configuration, it is possible to prevent the depletion of the liquid metal LM in the gaps between the bearings. The liquid metal LM forms a dynamic pressure sliding bearing together with the bearing surface of the stationary shaft 10 and the bearing surface of the rotor 20.

The anode target 50 is formed into a circular ring shape and is provided to be coaxial with the stationary shaft 10 and the bearing member 21. In one example, the anode target 50 is disposed to oppose the radial bearing surfaces S11a and S11b of the stationary shaft 10 in a direction that is orthogonal to the rotation axis a. In other words, a part of the radial bearing surfaces S11a and S11b is located in the region A opposing the anode target 50. The anode target 50 includes an anode target main body 51 and a target layer 52 provided on a part of the outer surface of the anode target main body 51. The anode target main body 51 is formed into a circular ring shape. The anode target main body 51 surrounds the outer circumference of the bearing member 21 and is fixed to the bearing member 21.

The anode target main body 51 is formed of molybdenum, tungsten, or an alloy of these materials. The melting point of the metal forming the target layer 52 is the same as, or higher than, the melting point of the metal forming the anode target main body 51. For example, the anode target main body 51 is formed of a molybdenum alloy, and the target layer 52 is formed of a tungsten alloy.

The anode target 50 is rotatable together with the rotor 20. When an electron collides with the target surface S52 of the target layer 52, a focal spot is formed on the target surface S52. The anode target 50 emits X-rays from the focal spot. That is, the anode target 50 generates X-rays by receiving electrons.

The cathode 60 is disposed to oppose the target layer 52 and be spaced apart from the target layer 52 of the anode target 50. The cathode 60 is attached to the inner wall of the vacuum tube 70. The cathode 60 includes a filament 61 as an electron emission source that emits electrons that irradiate the target layer 52.

The vacuum tube 70 is formed into a cylindrical shape. The vacuum tube 70 is formed from glass, ceramics, and metal. In the vacuum tube 70, the outer diameter of the part opposing the anode target 50 is greater than the outer diameter of the part opposing the tubular unit 24. The vacuum tube 70 has openings 71 and 72. The vacuum tube 70 is sealed, and accommodates the anode target 50 and the cathode 60, and fixes the stationary shaft 10. The interior of the vacuum tube 70 is maintained in a vacuum state (decompressed state).

In order to maintain the air tightness of the vacuum tube 70, the opening 71 is airtightly joined to one end of the stationary shaft 10 (the first small diameter unit 12), and the opening 72 is airtightly joined to the other end of the stationary shaft 10 (the second small diameter unit 13). The vacuum tube 70 fixes the first small diameter unit 12 and the second small diameter unit 13 of the stationary shaft 10. In other words, the first small diameter unit 12 and the small diameter unit diameter unit 13 function as a double-sided support sections for the bearing.

Note that the rotating anode X-ray tube 1 of this embodiment shown in FIG. 4 is configured as a double-sided support structure in which both ends of the stationary shaft 10 are fixed, but it may as well be configured as a single-sided support structure in which only one end of the stationary shaft 10 is fixed.

The stator coil 2 is provided to surround the outer side of the vacuum tube 70, while opposing the outer circumferential surface of the rotor 20, or more specifically, the outer circumferential surface of the tubular unit 24. The stator coil 2 is ring-shaped. The stator coil 2 generates a magnetic field that is applied to the tubular unit 24 (rotor 20), to rotate the rotor 20 and the anode target 50.

As described above, an X-ray tube assembly comprising the rotating anode X-ray tube 1 is formed.

In the operating state of the above-described X-ray tube assembly, the stator coil 2 generates a magnetic field that is applied to the rotor 20 (in particular, the tubular unit 24), so as to rotate the bearing member 21. Thus, the bearing member 21 and the anode target 50 as well rotate together. Further, a current is applied to the cathode 60, and a negative voltage is applied to the cathode 60, whereas a relatively positive voltage is applied to the anode target 50.

With this configuration, a potential difference is created between the cathode 60 and the anode target 50. The filament 61 emits electrons. These electrons are accelerated and collide with the target surface S52. With this configuration, a focal spot is formed on the target surface S52, and the focal spot emits X-rays when it collides with the electrons. The electrons that collide with the anode target 50 (thermionic electrons) are converted into X-rays, and the remainder is converted into thermal energy. The electron emission source of the cathode 60 is not limited to a filament, and may as well be a flat emitter, for example.

The heat generated by the anode target is transmitted from the target surface S52, through the interior of the target layer 52, the anode target main body 51, the bearing member 21, the liquid metal LM, and the stationary shaft 10 in this order. The heat transmitted to the stationary shaft 10 is transferred from the heat transfer unit 10a of the stationary shaft 10 to the cooling liquid L, and is emitted outside the sliding bearing 3 together with the cooling liquid L.

When the temperature of the bearing member 21, the liquid metal LM, and the stationary shaft 10 rises, the bearing surface (the radial bearing surfaces S11a, S11b, S21) undergoes a chemical reaction with the liquid metal LM. In this manner, a compound is formed on the bearing surface. Note that the rate at which compounds between the SKD11 and the liquid metal LM are formed is approximately 0.3 µm/h at 300°C, and the rate at which compounds are formed between tungsten carbide and the liquid metal LM is approximately 0.003 µm/h at 300°C. That is, even when at least one of the stationary shaft 10 and the bearing member 21 are formed of tungsten carbide, and the lubricant is a gallium-indium alloy or gallium-indium-tin alloy, the formation of the compounds can be suppressed. Note that when the suppression of the formation of the compound is considered important, it is preferable that both the stationary shaft 10 and the bearing member 21 be formed of tungsten carbide.

Further, when the temperature of the bearing member 21 rises, the bearing member 21 expands in the direction away from the stationary shaft 10, and the gap between the stationary shaft 10 and the bearing member 21 widens. When the gap between the stationary shaft 10 and the bearing member 21 widens, the bearing load decreases, and the risk of occurrence of vibration and cavitation increases.

Moreover, the X-ray tube assembly, together with the rotating stand 84, rotates and is subjected to a centrifugal acceleration CA in a direction perpendicular to the rotation axis a. When the Young's modulus of the stationary shaft 10 is low, the stationary shaft 10 deforms to bend in the same direction as that of the centrifugal acceleration CA.

Advantageous effects of the rotating anode X-ray tube 1 of the above-provided embodiment will be described.

According to the rotating anode X-ray tube configured as described above, the rotating anode X-ray tube 1 comprises a cathode 60, an anode target 50, a sliding bearing 3 including a stationary shaft 10 and a rotor 20, and a vacuum tube 70. The rotor 20 includes a bearing member 21 that is positioned to surround the stationary shaft 10. At least one of the stationary shaft 10 and the bearing member 21 are formed of tungsten carbide, silicon carbide, or titanium carbide.

With this configuration, when the stationary shaft 10 is formed of tungsten carbide, silicon carbide, or titanium carbide, the deformation by bending of the stationary shaft 10 due to the centrifugal acceleration CA of the stationary shaft 10 can be suppressed more than that of the case where the stationary shaft 10 is formed of SKD11.

Further, when the bearing member 21 is formed of tungsten carbide, silicon carbide, or titanium carbide, it is possible to prevent the gap between the stationary shaft 10 and the bearing member 21 from excessively widening compared to the case where the bearing member 21 is formed of SKD11. With this configuration, the occurrence of vibration and cavitation can be suppressed when the rotor 20 is rotating. Further, more heat can be transferred from the bearing member 21 to the stationary shaft 10, and therefore the cooling of the rotating anode X-ray tube 1 can be promoted.

When the materials of the stationary shaft 10 and the bearing member 21 are the same as each other, the linear expansion coefficients can be made the same. Therefore, when the temperatures of the stationary shaft 10 and the bearing member 21 substantially match each other, the gap between the stationary shaft 10 and the bearing member 21 can be maintained at a constant size.

If at least one of the stationary shaft 10 and the bearing member 21 are formed of tungsten carbide, and the lubricant is a gallium-indium alloy or gallium-indium-tin alloy, the formation of compounds can be suppressed.

From the descriptions above, according to this embodiment, it is possible to obtain a rotating anode X-ray tube that can maintain good rotation of the rotor 20.

### (Modified Example)

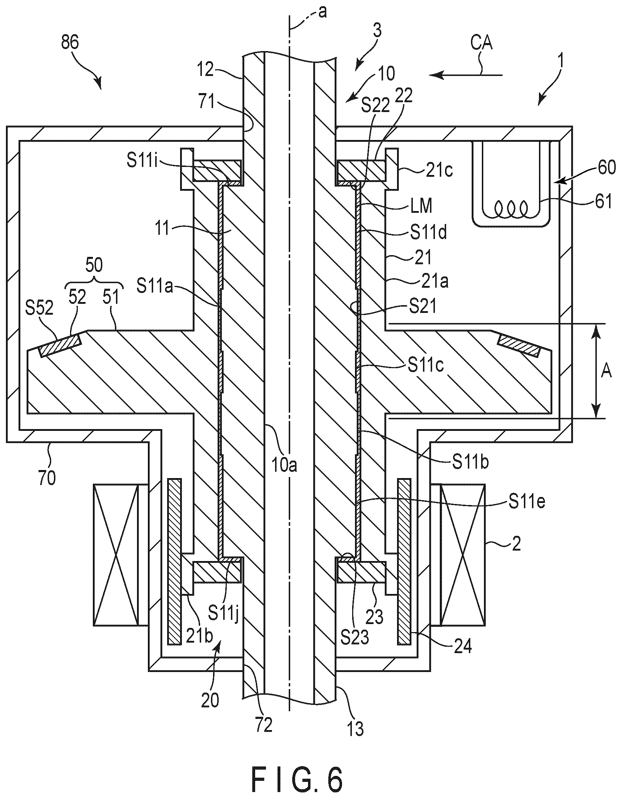

Next, a modified example of the rotating anode X-ray tube 1 according to the embodiment described above will be explained. The rotating anode X-ray tube 1 is configured in a way similar to that of the above-described embodiment, except for the configuration explained in this modified example. FIG. 6 is a cross-sectional view showing the modified example of the rotating anode X-ray tube of the embodiment described above.

As shown in FIG. 6, the anode target 50 is positioned to surround the rotor 20 (or more specifically, the bearing member 21). The anode target 50 includes an anode target main body 51 and a target layer 52 that is provided on the outer surface of the anode target main body 51 so as to oppose the cathode 60.

The anode target main body 51 is formed of the same material as that of the bearing member 21 and is formed continuously to be integrated with the bearing member 21 as one body. In other words, the anode target main body 51 and the bearing member 21 are not joined together. The integrated unit formed by the bearing member 21 and the anode target main body 51 can be manufactured by casting, cutting or the like.

According to the modified example of the rotating anode X-ray tube 1 according to the above-described embodiment, the anode target main body 51 is formed to be integrated with the bearing member 21 as one body. With this configuration, more of the heat generated by the anode target 50 can be transferred to the stationary shaft 10 as compared to the case where the anode target main body 51 is joined to the bearing member 21.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. A rotating anode X-ray tube comprising:
a cathode (60) which emits electrons;
an anode target (50) which generates X-rays upon receiving the electrons;
a sliding bearing (3) including a rotor (20) coupled with the anode target (50) and extending along a rotation axis (a), a stationary shaft (10) supporting the rotor (20) so as to be rotatable, and a lubricant held between the rotor (20) and the stationary shaft (10); and
a vacuum tube (70) which accommodates the cathode (60) and the anode target (50) and fixes the stationary shaft (10),
**characterized in that**:
the rotor (20) includes a bearing member (21) formed to extend along the rotating axis (a) and positioned to surround the stationary shaft (10), and
at least one of the stationary shaft (10) and the bearing member (21) are formed of tungsten carbide, silicon carbide, or titanium carbide.

2. The rotating anode-type X-ray tube of claim 1, **characterized in that**:
the anode target (50) is positioned to surround the bearing member (21) and comprises an anode target main body (51) and a target layer (52) provided on an outer surface of the anode target main body (51) and opposing the cathode (60), and
the anode target main body (51) is formed of a material the same as that of the bearing member (21) and is formed continuously to be integrated with the bearing member (21).

3. The rotating anode-type X-ray tube of claim 1, **characterized in that**:
a material of the stationary shaft and a material of the bearing member are the same as each other.

4. The rotating anode-type X-ray tube of claim 3, **characterized in that**:
the anode target (50) is positioned to surround the bearing member (21) and includes an anode target main body (51) and a target layer (52) provided on an outer surface of the anode target main body (51) and opposing the cathode (60), and
the anode target main body (51) is formed of a material the same as that of the bearing member (21) and is formed continuously so as to be integrated with the bearing member (21).

5. The rotating anode-type X-ray tube of claim 1, **characterized in that**:
the lubricant is a gallium-indium alloy or gallium-indium-tin alloy.

6. The rotating anode-type X-ray tube of claim 5, **characterized in that**:
the anode target (50) is positioned to surround the bearing member (21) and includes an anode target main body (51) and a target layer (52) provided on an outer surface of the anode target main body (51) and opposing the cathode (60), and
the anode target main body (51) is formed of a material the same as that of the bearing member (21) and is formed continuously so as to be integrated with the bearing member (21).
